# EUROPEAN PATENT SPECIFICATION

(11) **EP 4 359 038 B1**
(45) Date of publication and mention of the grant of the patent: **18.11.2026**
(21) Application number: 22735392.7
(22) Date of filing: 21.06.2022
(51) Int. Cl.: A61M 5/24, A61M 5/315, A61M 5/50

(54) **A SINGLE DOSE INJECTION DEVICE**
MEDIZINISCHE INJEKTIONSVORRICHTUNG
DISPOSITIF D'INJECTION MÉDICALE

(30) Priority: 22.06.2021 EP 21180787
(43) Date of publication of application: 01.05.2024
(73) Proprietor: Novo Nordisk A/S, 2880 Bagsværd (DK)
(72) Inventor: MORTENSEN, Rasmus, Døssing, 2880 Bagsværd (DK); ELLINGER , Beatrice, Angel, 2880 Bagsværd (DK); WINDUM, Jesper, Peter, 2880 Bagsværd (DK); KIILERICH, Ebbe, 2880 Bagsværd (DK); KRONBORG, Nikolaj, Thorsø, 2880 Bagsværd (DK)
(86) International application number: PCT/EP2022/066818
(87) International publication number: WO 2022/268771

(56) References cited:
- US-A- 2 753 867
- US-A1- 2004 162 528
- US-A1- 2016 008 548
- US-A1- 2018 140 776

## Description

### THE TECHNICAL FIELD OF THE INVENTION:

The invention relates to a single dose injection device for expelling one dose of a predetermined fixed volume of a liquid drug. More particular the invention relates to an injection device which is pre-filled by the manufacturer of the injection device by use of a pre-filled cartridge and designed for manually expelling one single fixed dose of a predetermined volume of liquid drug from the encapsulated cartridge and preferably by one stroke of an axially movable piston rod moving translational inside the cartridge.

The invention further relates to a method of assembling such single dose injection device.

### DESCRIPTION OF RELATED ART:

Syringes used to expel the full content of a cartridge in one stroke has been known for decades. An example of such syringe is disclosed in US 2,753,867. Here an exchangeable cartridge containing a liquid drug is inserted into a tubular housing structure and a threaded nut element guiding a piston rod is screwed into the housing structure to secure the cartridge axially relatively to the housing structure. The content of the cartridge is expelled by moving a plunger in the distal direction inside the cartridge. The axial movement of the plunger is performed by the user manually pushing the piston rod in the distal direction. The full injectable volume contained in the cartridge is expelled as one single dose through a double pointed needle assembly attached to a needle interface provided distally on the housing structure. The forward movement of the piston rod is first stopped when the plunger inside the cartridge reaches the distal neck part of the cartridge hence the proximal stop is not fixed but depend on the physical shape of the cartridge and the resiliency of the plunger. The volume expelled as the plunger moves through the cartridge hence very much depend on the physical tolerances of the cartridge and the tolerances in the filling process.

A different example of an automatic spring-operated injection device wherein a cartridge can be used is provided in EP 3,628,352. In this example, the cartridge used has a difference in the fill volume due to tolerances and therefore also a different axial position of the plunger prior to use. Therefore, once the cartridge is mounted in the housing structure, a gap can exist between the plunger and the piston rod. When injecting, the piston rod may move in this free space without actually moving the plunger inside the cartridge which means that the axial movement of the piston rod is not converted directly into a similar movement of the plunger which causes the volume of the expelled dose to vary from cartridge to cartridge depending on the tolerances determining the position of the plunger in the individual cartridge. To compensate for this, the automatic firing mechanism can be adjusted in its relative axial position during assembly of the injection device.

An example of a drug delivery device with an adjustable nut element is provided in US 2016/008548 A1. In this example the drug delivery device has an adjustment member in form of a nut that guides a piston rod which extends through the adjustment member. The adjustment member has an internal thread that is in contact with an external thread of the piston rod. The adjustment member has further a fixing feature that after adjustment engages with a housing of the drug delivery device and prevents rotation of the adjustment member relative to the housing. During assembly, when a cartridge is inserted to a distal part of the housing, by rotation of the adjustment member the piston rod is moved distally into contact with a bung of the cartridge. Once the piston rod contacts the bung further advancement of the piston rod is not possible and further rotation of the adjustment member brings the adjustment member proximally into a position in which it engages with the housing.

A more traditional manual multi-dose insulin injection device is disclosed in US

2018/0140,776. In this injection device a piston rod is rotated in a threaded nut element to move helically in the distal direction during injection to thereby press out an individually set dose. During assembly of this injection device it is possible to axially position the nut element in relation to the housing.

### DESCRIPTION OF THE INVENTION:

It is henceforth an object of the present invention to provide a solution wherein the volume expelled when expelling the single fixed dose is not dependent on the above explained tolerances.

This is preferably done by moving the piston rod forward a predetermined travel distance in one single stroke of the piston rod and to make the physical position of this travel distance in relation to the housing structure and thus the cartridge embedded in the housing structure variable for each individual injection device.

The invention is further defined in claim 1. Advantageous embodiments are defined in the dependent claims. Accordingly, in one aspect of the present invention, a single dose injection device for delivering a predetermined volume of a liquid drug, comprises:
a housing structure holding a loaded cartridge embedded in and axially locked to the housing structure and containing the liquid drug to be expelled and which cartridge has a distal end sealed by a pierceable septum and a proximal end closed by a movable plunger having a distal front surface,
a piston rod structure comprising a piston rod and a piston rod foot, which piston rod structure is translationally movable in the distal direction in relation to the housing structure to thereby move the plunger in the distal direction inside the cartridge, the plunger being movable from a first position to a second position, wherein
the predetermined volume of liquid drug to be ejected is defined by an inner surface of the cartridge and the length the distal front surface of the plunger is moved during movement of the plunger from the first position to the second position, and wherein a nut element guiding the piston rod structure is adjustable coupled to the housing structure.

Further, according to the invention, the piston rod structure and the nut element are provided with engagement means for releasable securing the piston rod structure to the nut element in a start position of the piston rod structure in which start position, the piston rod structure abuts the plunger, at least in use,
and wherein a stop position of the piston rod structure is defined by stop means on the piston rod operationally engaging the nut element, such that a travel distance of the piston rod structure is defined as the axial distance the piston rod structure is translationally movable when moving the plunger from its start position to its stop position during dose ejection, and wherein the axial position of the nut element relatively to the housing structure is individually adjustable for each individual injection device during assembly of the individual injection device to thereby adjust the axial position of the travel distance of the piston rod structure to an adjusted position in dependency of the position of the plunger in the individual cartridge.

The travel distance of the piston rod is thus the distance the piston rod is movable from its start position to its stop position. However, it is important that the travel distance is transformed one-to-one to plunger movement all though the plunger has a variable position inside each individual cartridge. This is achieved by guiding the piston rod in a nut element which is adjustable in relation to the housing structure. It is hence possible, for each individual injection device to adjust the physical position of the travel distance. The position of the travel distance is preferably adjusted such that the piston rod structure (including the piston rod foot) abuts the plunger in the start position of the piston rod.

Preferably, the piston rod foot abut the plunger in a situation of use. Due to temperature variations, the abutment is not necessarily fully established when the injection device is in storage. However, as a starting position, when in use, the piston rod foot abuts the plunger.

The actual physical position of the start position is preferably determined by the physical design of the nut element. However, the distance between the start position and the stop position is decisive for the length the piston rod moves during ejection and the position of this distance, the travel distance, is adjustable for each individual injection device.

All though the travel distance is a fixed distance, the position of the travel distance in relation to the housing structure is, as herein explained, adjustable. As the cartridge is fixed to the housing structure, the position of the travel distance in relation to the cartridge and to the plunger inside the cartridge are also adjustable.

The nut element and hence the position of the travel distance are thus individually adjustable for each individual injection device and the position of the travel distance can be adjusted to accommodate the individual position of the plunger inside each cartridge.

The piston rod is coupled to the nut element in a position defining the start position but in a releasable coupling such that the piston rod can be moved out of the start position to start the ejection. Further, the stop position for the piston rod is defined by a stop surface on the piston rod. In the stop position this stop surface engages and abuts against the nut element.

The distance between the start position and the stop position is the travel distance that the piston rod travels in relation to the nut element and to the housing structure including the cartridge during one single ejection and could also be referred to as the length of the stroke of the piston rod.

The piston rod structure is releasable secured to the nut element by releasable engagement means. These releasable engagement means secures the piston rod in a releasable position from which the piston rod can be released simply by applying a pressure on to the proximal end of the piston rod. In one example, the engaging means between the piston rod and the nut element comprises a number of radial arms provided on either the piston rod or the nut element engaging the other of the piston rod or the nut member.

In a preferred example, the radial arms are provided on the piston rod whereas the nut element is provided with an angled front which is engaged by the radial arms when the piston rod is in its start position. The angled front is preferably sloped in the proximal position to better prevent the piston rod from movement in the proximal direction when assembled.

Alternatively, the engagement means between the piston rod and the nut element comprises a number of further radial arms provided on the piston rod frictionally engaging a recessed inner surface on the nut element in the start position of the piston rod.

In a different example the engaging means between the piston rod and the nut element comprises a number of radial gripping teeth provided on either the piston rod or the nut element engaging the other of the piston rod or the nut member. In this example, the radial gripping teeth are preferably provided on the piston rod and the nut element is provided with an angled surface which is engaged by the radial gripping teeth when the piston rod is in its start position. The gripping teeth can have any desired size and shape.

The physical location of the start position is determined by the physical design of the nut element which in one example is provided with a proximal flange which defines the start position for the piston rod and which proximal flange engages with the stop means on the piston rod in the stop position.

The adjustment of the nut element in relation to the housing structure can be envisage in a number of different ways. It could be a strictly linear adjustment, or it could be a helical adjustment. In a preferred example, the nut element is preferably axially adjusted in relation to the housing structure in a threaded connection between the nut element and the housing structure.

Following the adjustment, the nut element can be secured to the housing structure in a number of different ways. In one example, the nut element and the housing structure could be welded together e.g. by laser welding. In a different example a ratchet interface could be used which specifically could be a one-way ratchet.

In one example the housing structure is provided with a thread on an inner surface engaging a corresponding thread on an outer surface of the nut element. These threads can be either full threads or segments of threads. In one example, the pitch of the threads are configured such that the nut element is moved in the distal direction when the nut element is rotated anti clockwise in the thread connection to thereby obtain the adjusted position.

In order to maintain the nut element in the adjusted position, the flanges of the thread can be provided with axial friction knobs. These knobs can have any desired size and shape necessary to hold the nut element from rotation in the opposite direction and away from the adjusted position. As an alternative to, or as an addition to the axial friction knobs a ratchet interface can be provided. Such ratchet interface could be formed as one or more flexible teeth operating in a toothed rib such that rotation is allowed in both rotational directions. To further prevent the nut element from unscrewing the ratchet interface can be a one-way ratchet interface provided between the nut element and the housing structure. Such a one-way ratchet interface can operate together with the described friction knobs but can also operate without the friction knobs.

Such one-way ratchet can in one example be provided as a flexible ratchet carrier provided on one of the nut element or the housing structure and an axial rib structure provided the other of the nut element and the housing structure. The flexible ratchet carrier thus engages with the axial rib structure to form the one-way ratchet. For this purpose, the ratchet carrier can be provided with one or more teeth for the actual engagement. The flexible ratchet carrier is preferably formed as an arm or as a beam construction. The nut element and the housing structure are thus irreversible connected in the assembled state. However, the flexible ratchet carrier could as mentioned be provided as a ratchet interface allowing rotation in both directions.

In a second aspect of the present invention, a method of assembling a single dose injection is provided. The method comprises a single dose injection device as defined in the claims wherein the method comprises the step of individually adjusting the axial position of the nut element for each individual injection device during assembly to thereby adjust the axial position of the travel distance of the piston rod structure relatively to the housing structure to an adjusted position wherein the piston rod structure abuts the plunger.

Hence, for each individual injection device the position of the nut element is adjusted to an adjusted position wherein the piston rod structure i.e. the piston rod or the piston rod foot abuts the plunger inside the cartridge such that the stroke of the piston rod is transferred to an identical linear movement of the plunger. By having this adjustability of the nut element and thus the travel distance of the piston rod it can be prevented that some of the piston rod movement is done in the air-gap space occasionally existing between the plunger and the piston rod structure due to the variable position of the plunger in the individual cartridge.

In a third aspect the invention relates to a plurality of single dose injection devices for delivering a predetermined volume of a liquid drug according to any of the apparatus claims and wherein each single dose injection device in the plurality has been individually assembled and adjusted according to the method claim.

### DEFINITIONS:

An **"injection pen"** or **"pen for injection"** is typically an injection apparatus having an oblong or elongated shape somewhat like a pen for writing. Although such pens usually have a tubular cross-section, they could easily have a different cross-section such as triangular, rectangular or square or any variation around these geometries.

The term **"Needle Cannula"** is used to describe the actual conduit performing the penetration of the skin during injection. A needle cannula is usually made from a metallic material such as e.g. stainless steel and connected to a hub to form a complete injection needle also often referred to as a **"needle assembly".** A needle cannula could however also be made from a polymeric material or a glass material. The hub also carries the connecting means for connecting the needle assembly to an injection apparatus and is usually moulded from a suitable thermoplastic material. The **"connection means"** could as examples be a luer coupling, a bayonet coupling, a threaded connection or any combination thereof.

The term **"Needle unit"** is used to describe one single needle assembly carried in a container. Such container usually has a closed distal end and an open proximal end which is sealed by a removable seal. The interior of such container is usually sterile such that the needle assembly is ready to use. Needle units specially designed for pen injection systems are defined in ISO standard No. 11608, part 2, and are often referred to as **"pen needles".** Pen needles are usually double pointed having a front-end for penetrating through the skin of a user and a back-end for penetrating into the cartridge containing the drug such that liquid communication is established during injection.

As used herein, the term **"Liquid drug"** is meant to encompass any drug-containing flowable medicine capable of being passed through a delivery means such as a hollow needle cannula in a controlled manner, such as a liquid, solution, gel or fine suspension. Representative drugs include pharmaceuticals such as peptides, proteins (e.g. insulin, insulin analogues and C-peptide), and hormones, biologically derived or active agents, hormonal and gene-based agents, nutritional formulas and other substances in both solid (dispensed) or liquid form.

**"Cartridge"** is the term used to describe the container actually containing the drug which are often referred to as the primary packing. Cartridges are usually made from glass but could also be moulded from a suitable polymer. A cartridge or ampoule is preferably sealed at one end by a pierceable membrane referred to as the **"septum"** which can be pierced e.g. by the non-patient end of a needle cannula. Such septum is usually self-sealing which means that the opening created during penetration seals automatically by the inherent resiliency once the needle cannula is removed from the septum. The opposite end of the cartridge is typically closed by a movable **"plunger"** which is a piston-like element made from rubber or a suitable polymer. The plunger is during use slidable moved inside the cartridge preferably in a distal direction. The space between the pierceable membrane and the movable plunger holds the liquid drug which is pressed out as the plunger decreased the volume of the space holding the liquid drug. The cartridges used for both pre-filled injection devices and for durable injections devices are typically factory filled by the manufacturer with a predetermined volume of a liquid drug. A large number of the cartridges currently available contains either 1,5 ml or 3 ml of liquid drug.

Since a cartridge usually has a narrower distal neck portion into which the plunger cannot be moved not all of the liquid drug contained inside the cartridge can actually be expelled. The term **"initial quantum"** or **"substantially used"** therefore refers to the injectable content contained in the cartridge and thus not necessarily to the entire content.

By the term **"Pre-filled injection device"** is meant an injection device in which the cartridge containing the liquid drug is permanently embedded in the injection device such that it cannot be removed without permanent destruction of the injection device. Once the pre-filled amount of liquid drug in the cartridge is used, the user normally discards the entire injection device. Usually the cartridge which has been filled by the manufacturer with a specific amount of liquid drug is secured in a cartridge holder which is then permanently connected in a housing structure such that the cartridge cannot be exchanged.

This is in opposition to a **"Durable injection device"** in which the user can himself change the cartridge containing the liquid drug whenever it is empty. Pre-filled injection devices are usually sold in packages containing more than one injection device whereas durable injection devices are usually sold one at a time. When using pre-filled injection devices an average user might require as many as 50 to 100 injection devices per year whereas when using durable injection devices one single injection device could last for several years, however, the average user would require 50 to 100 new cartridges per year.

This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1A-B: show perspective views of the injection device with (figure 1A) and without (figure 1B) the protective cap mounted.
- Figure 2: show an exploded view of the injection device.
- Figure 3: show a cross-sectional view of the injection device when assembled and prior to expelling the single fixed dose.
- Figure 4A-B: show two different views of the housing structure. In figure 4B, the housing structure is cut open along the longitudinal centre axis.
- Figure 4C: show a cross-sectional view of another example of the injection device.
- Figure 4D: show a section of the injection device in figure 4C.
- Figure 5A-B: show two different views of the nut element. In figure 5B, the nut element is cut open along the longitudinal centre axis.
- Figure 5C: show an enlarged view of the distal part of the nut element in an alternative embodiment.
- Figure 6A-C: show different views of the piston rod. Figure 6B is an enlarged view of the encircled section of figure 6A.
- Figure 7: show a perspective view of the injection button.
- Figure 8: show a cross-sectional view of a pen needle suitable for the disclosed injection device.
- Figure 9A: show a cross-sectional view of the nut element mounted in the housing structure.
- Figure 9B: show a cross-sectional view of the injection device assembled and ready to dose.
- Figure 9C: show a cross-sectional view of the injection device after dosing.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter-clockwise" or similar relative expressions are used, these only refer to the appended figures and not necessarily to an actual situation of use. The shown figures are schematic representations for which reason the configuration of the different structures as well as their relative dimensions are intended to serve illustrative purposes only.

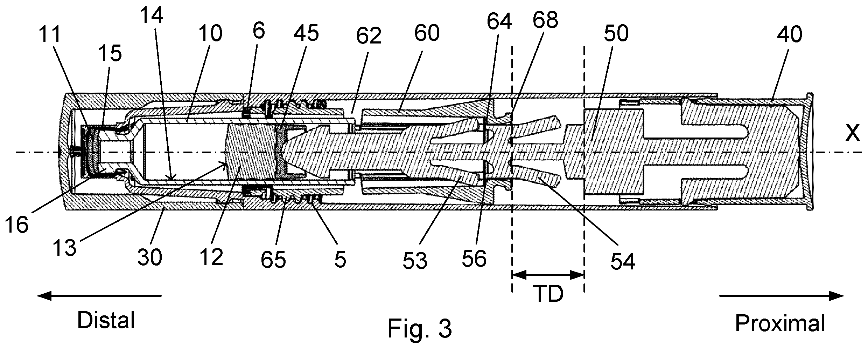

In that context it may be convenient to define that the term "distal end" in the appended figures is meant to refer to the end of the injection device supporting the injection needle, whereas the term "proximal end" is meant to refer to the opposite end carrying the injection button as indicated on figure 3. Distal and proximal is meant to be along an axial orientation extending along the longitudinal axis (X) of the injection device as also shown in figure 3.

When referring to clock-wise and anti or counter clock-wise in the following examples it is understood that the injection device is viewed from a position distal to the injection device. Clock-wise is thus a rotation following the arms on an ordinary clock and counter clock-wise is a rotation in the opposite direction.

To explain the various movements which take place in the injection device described in the example, the following terminology are used throughout the following detailed description;
"Translational movement" is meant to be a strictly linear movement without any rotation.

"Rotational movement" is any movement of rotation around a centre which centre can be a centre point i.e. in one planar or a centre axis i.e. having a longitudinal extension.

"Axial movement" means any movement in an axial direction. Such movement can be a strictly translational movement or include a rotational movement which thus makes it a "Helical movement" as this is meant to be an axial movement combined with a rotational movement.

"Telescopic" is meant to cover the situation in which a movable element moves out from, and/or into, a base element. The telescopic movement can be either translational or include a rotation thus making the telescopic movement helical.

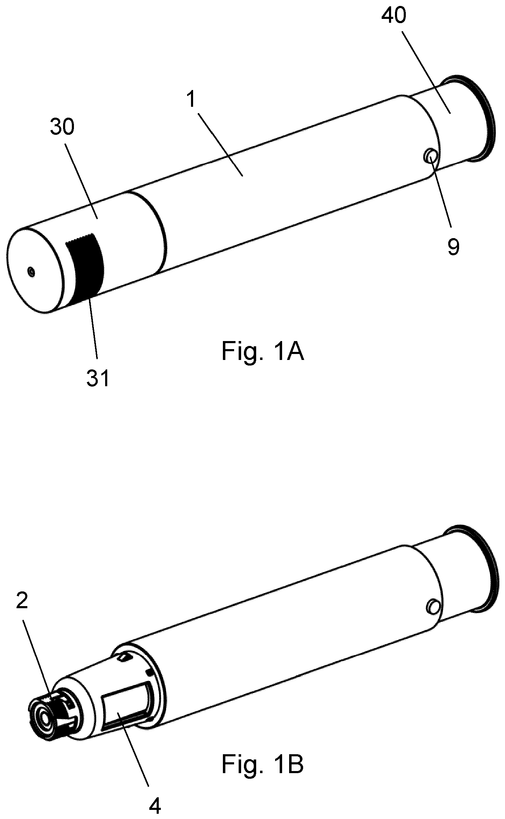

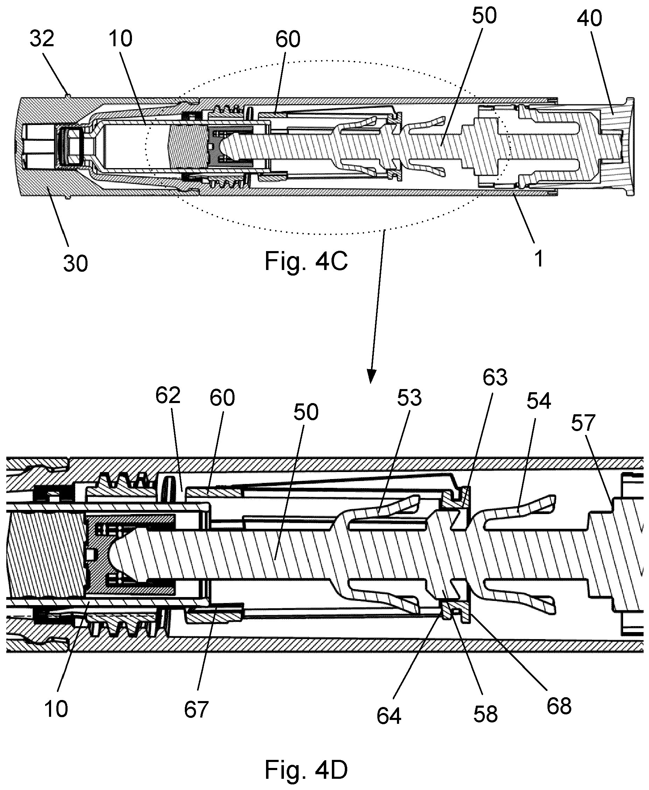

Figure 1A**-B** discloses the injection device according to the invention. The housing structure 1 is distally provided with a protective cap 30 and proximally provided with an injection button 40. In figure 1B, the protective cap 30 covering the distal part of the injection device has been removed as it is usually done prior to performing an injection. As best seen in figure 1A, the protective cap 30 has an area 31 with a different surface texture or a rib-like structure 32 (best seen in figure 4C) enabling the user to better grip the protective cap 30 when removing it. The housing structure 1 is preferably provided with an anti-roll feature 9 such that the injection device does not roll when laying on a flat or slightly tilting surface such as a table or the like.

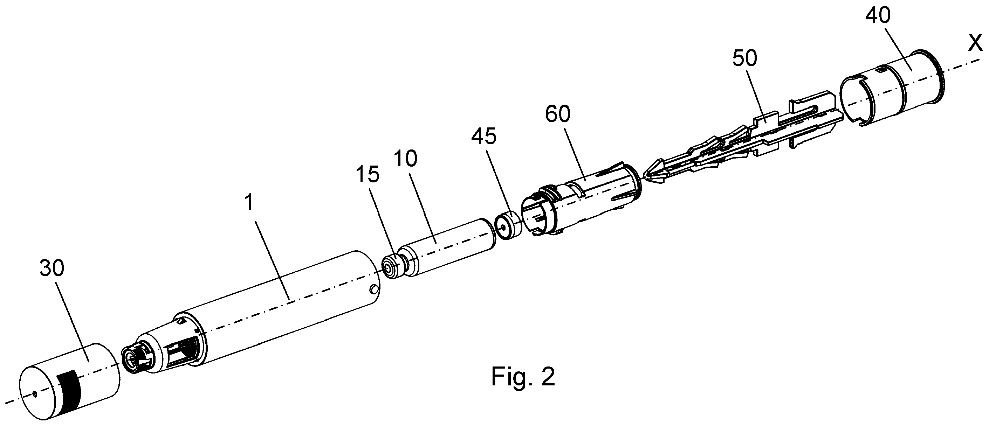

As seen in **figure 2** and in **figure 3** the injection device comprises the housing structure 1 which can be formed from one or more parts. The housing structure 1 axially fixates a cartridge 10 containing the liquid drug to be injected and is distally provided with a needle interface 2 for securing a needle assembly 20 to the housing 1. The distal needle interface 2 is preferably covered by the removable protective cap 30 when the injection device is not in active use. Proximally, the housing 1 is provided with a push-like injection button 40 which is operable by the user to perform the injection. The injection button 40 is directly coupled to the piston rod 50 such that any translation of the injection button 40 in the distal direction is immediately transformed to a similar translation of the piston rod 50 as will be explained.

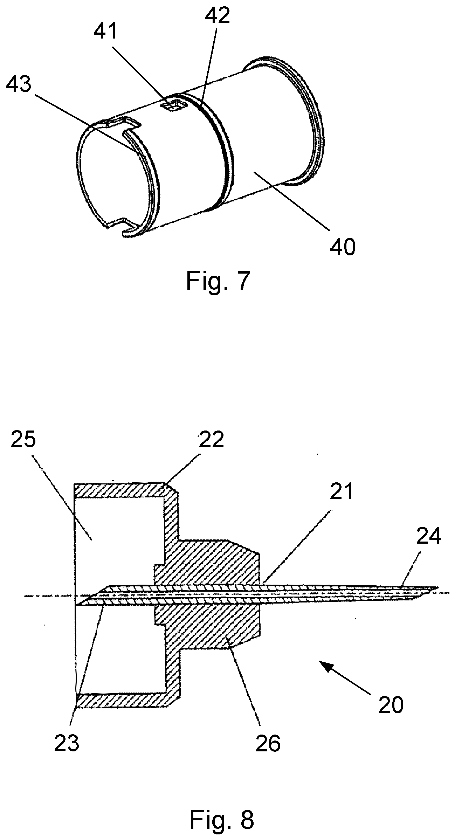

The needle assembly 20 used is a standard pen needle as disclosed in **figure 8****.** In such well-known pen-needle, a needle cannula 21 is secured in a needle hub 22 such that a proximal part 23 of the needle cannula 21 points in the proximal direction to penetrate into the cartridge 10 when the needle assembly 20 is attached to the needle interface 2. The opposite distal part of the needle cannula 21 is provided with a sharp distal point 24 which is able to penetrate the skin of a user during injection. Proximally the needle hub 22 forms an enclosure 25 surrounding the proximal part 23 of the needle cannula 21. This enclosure 25 usually carries means for connecting the needle hub 22 to an injection device such that the proximal part 23 of the needle cannula 21 penetrates into the cartridge 10 when the needle assembly 20 is connected. Distally the needle hub 22 extends into a tower 26 supporting the needle cannula 21.

In one example, the needle cannula 21 could be a gauge 29 (G29) needle cannula and the length of the distal part of the needle cannula 21 being inserted into the skin could be 4 mm when measured from the needle hub 22 to the sharp distal point 24. The distal part of the needle cannula 21 could further have a conical shape.

Inside the housing structure 1, the cartridge 10 is positioned as disclosed in figure 3. The cartridge 10 is distally provided with a pierceable septum 11 through which the needle cannula 21 penetrates when mounted. At the opposite proximal end, the cartridge 10 is closed by a movable plunger 12 preferably made from a suitable polymer and having a distal surface 13 which is in contact with the liquid drug in the cartridge 10. The space between the distal surface 13 of the movable plunger 12, the septum 11 and the inner surface 14 of the cartridge 10 contains the liquid drug as best seen in figure 3. The volume to be expelled during injection is defined by the inner surface 14 of the cartridge 10 and the axial length the distal surface 13 of the movable plunger 12 is moved during dose expelling.

Distally the septum 11 is secured to the cartridge 10 by a metal cap 15 which is bended around a neck 16 of the cartridge 10 as it is generally know from cartridges.

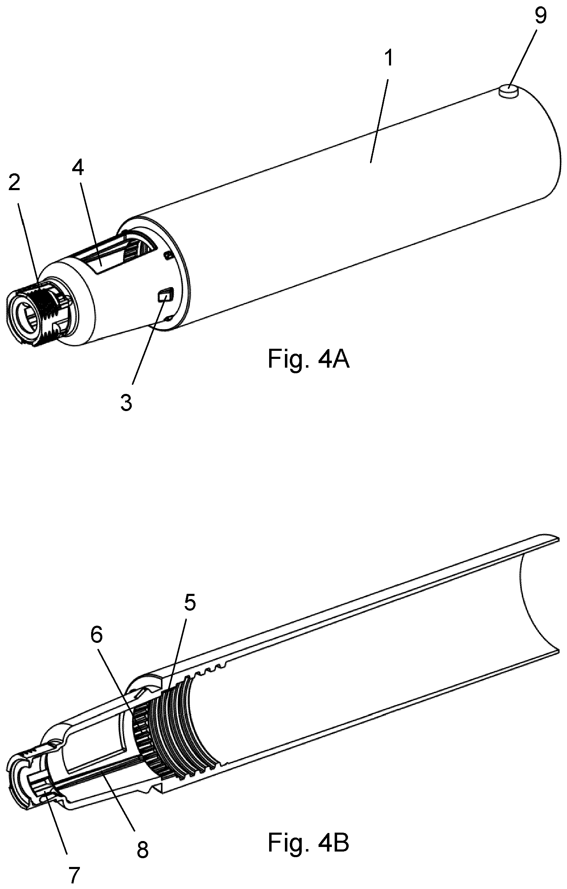

The housing structure 1 disclosed in figure 4A**-B** is in one example distally provided with a plurality of crush ribs 7 or the like which are designed to fixate the cartridge 10 to the housing structure 1. Once the cartridge 10 is press fitted into the housing structure 1 during assembly, the deformation of these crush ribs 7 against the metal cap 15 on the cartridge 10 secures the cartridge 10 to the housing structure 1. In a different example, alternative means can be provided to secure the cartridge 10 relatively to the housing structure 1. The cartridge 10 only needs to be fixed to the housing structure in the axial direction. In a preferred alternative, the housing structure 1 is provided with a number of inwardly pointing knobs which grip behind the neck 16 of the cartridge 10 to thereby secure the cartridge 10 to the housing structure in the axial direction. Such knobs could be combined with the disclosed crush ribs 7.

A different alternative of fixating the cartridge 10 to the housing structure 1 is disclosed in **figure 4C****.** In this example, the injection device comprises the same elements as in the first example as illustrated in figure 4C, namely, a housing structure 1 with a protective cap 30 having ribs 32, a cartridge 10, an injection button 40, a piston rod 50 and a nut element 60.

**Figure 4D** discloses an enlarged part from figure 4C wherein the nut element 60 is provided with a number of inwardly pointing longitudinal crush flanges 67 which engages the proximal end of the cartridge 10 when the nut element 60 is threaded to the housing structure 1 as will be explained. The cartridge 10 is thus axially locked between the housing structure 1 and the crush flanges 67 on the nut element 60 rendering the cartridge 10 axially locked to the housing structure 1. The crush flanges 67 are hence partly crushed by the proximal end of the cartridge 10 as the nut element 60 is moved into engagement with the housing structure 1. In one example four such crush flanges 67 are provided but any number can be used. In the disclosed example, the crush ribs 7 shown in figure 4B and/or the above discussed inwardly pointing knobs are not necessarily needed but could be applied if wanted.

During injection, the plunger 12 is moved in the distal direction inside the cartridge 10 by a piston rod 50 which in the disclosed examples distally are provided with a separate piston rod foot 45 to better distribute the forces onto the plunger 12. The piston rod foot 45 could in one example be moulded integral with the piston rod 50.

The piston rod 50 engages, and is guided by, a nut element 60 which is axially adjustable relatively to the housing structure 1 as will be explained.

As best seen in figure 4A, the housing structure 1 is on the outer surface provided with one or more protrusions 3 which secures the protective cap 30 in a releasable coupling. Further, the housing structure 1 has a window 4 through which a user can inspect the content of the cartridge 10. Most distally, the housing structure 1 is provided with the needle interface 2 for connecting the needle assembly 20 to the housing structure 1 which in the disclosed example is a combination of a threaded interface and a bayonet interface, however any kind of needle interface means can be used.

Besides the crush ribs 7 provided on the inner surface of the housing structure 1, a number of longitudinal ribs 8 for supporting the cartridge 10 are also provided. Further, the housing structure 1 has its inner surface provided with an inner thread 5 for securing the nut element 60 as will be explained. Distally to the inner thread 5, a toothing comprising a number of axial ribs 6 are provided which engages with the nut element 60.

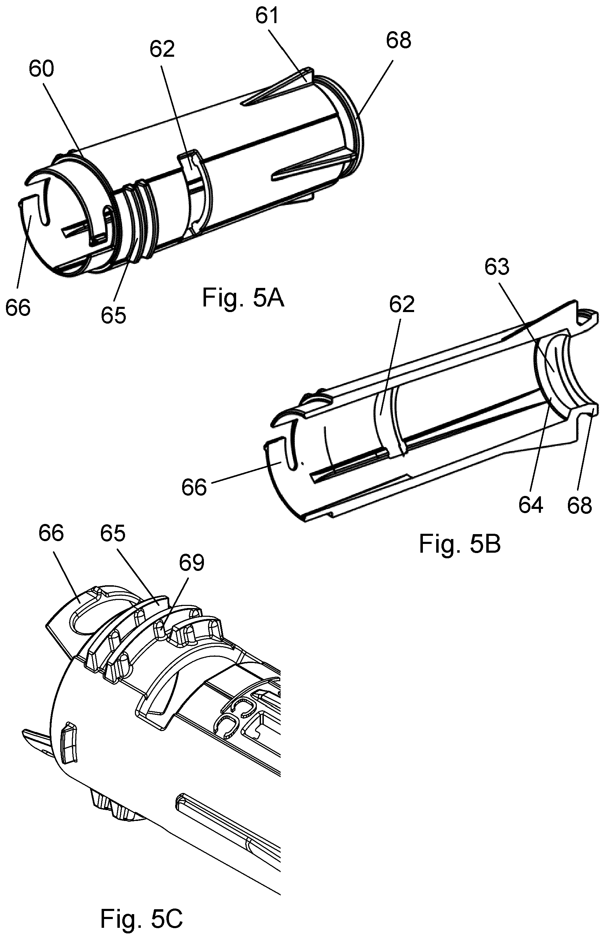

The nut element 60 is disclosed in figure 5A-B and is on the outer surface provided with outer thread segments 65 for engaging the inner thread 5 in the housing structure 1 such that the nut element 60 can be rotated relatively to the housing structure 1 in a rotation which moves the nut element 60 axially in relation to the housing structure 1. Any number of thread segments 65 can be provided as disclosed, alternatively, one full thread can be provided.

In one embodiment disclosed in **figure 5C****,** the thread 5 and/or the thread segments 65 can be provided with axial friction knobs 69 which engages to further secure the engagement between the two threads (inner thread 5 of the housing structure 1 and thread segments 65 on the nut element 60). The friction knobs 69 are axially provided on the radial flange of the threads 5, 65 and thus helps to frictional secure the housing structure 1 and the nut element 60 to each other. The friction knobs 69 as disclosed in figure 5C can be provided in any size and number required. The presence of these friction knobs 69 also makes it possible to adjust the engagement between the nut element 60 and the housing structure 1 in very fine increments depending on the rotational distance between the individual friction knobs 69.

Distally, the nut element 60 is provided with a number of flexible ratchet carriers 66 which flexes in the radial direction and engages the axial ribs 6 in the housing structure 1 such that the nut element 60 can only be rotated relatively to the housing structure 1 in one rotational direction. These flexible ratchet carriers 66 can be provided as flexible arms as disclosed in figure 5A-B or as a beam structure as disclosed in figure 5C or any other suitable structure. In the disclosed example, the nut element 60 can only be rotated counter clock-wise (when viewed from a distal position) and the thread interface 5, 65 (between inner thread 5 on the housing structure 1 and the outer thread segments 65 on the nut element 60) has a pitch direction such that rotation of the nut element 60 in the allowed counter clock-wise direction moves the nut element 60 in the distal direction inside the housing structure 1.

However, the flexible ratchet carriers 66 need not be executed as a one-way ratchet but can also be shaped such that rotation is possible in both rotational directions.

On the outer surface, the nut element 60 is provided with a number of outwardly pointing support flanges 61 which lie against the inner surface of the housing structure 1. As best seen in figure 5A-B , four such outwardly pointing support flanges 61 are provided but any number can be used.

The nut element 60 is further provided with a number of radial openings 62 which can be aligned with the most proximal end of the cartridge 10 when mounted as best seen in figure 3. However, if the assembly method of figure 4C-D is used, these radial openings 62 can be provided at a different location as seen in figure 4C-D. At the proximal end, the nut element 60 is provided with a recessed inner surface 63 to accommodate the piston rod 50. This recessed inner surface is distally provided with an angled front 64 sloping towards the inner surface of the nut element 60 and proximally the nut element 60 terminates in a proximal flange 68.

Further, the inner surface of the nut element 60 is provided with a number of longitudinal support ribs which supports the cartridge 10.

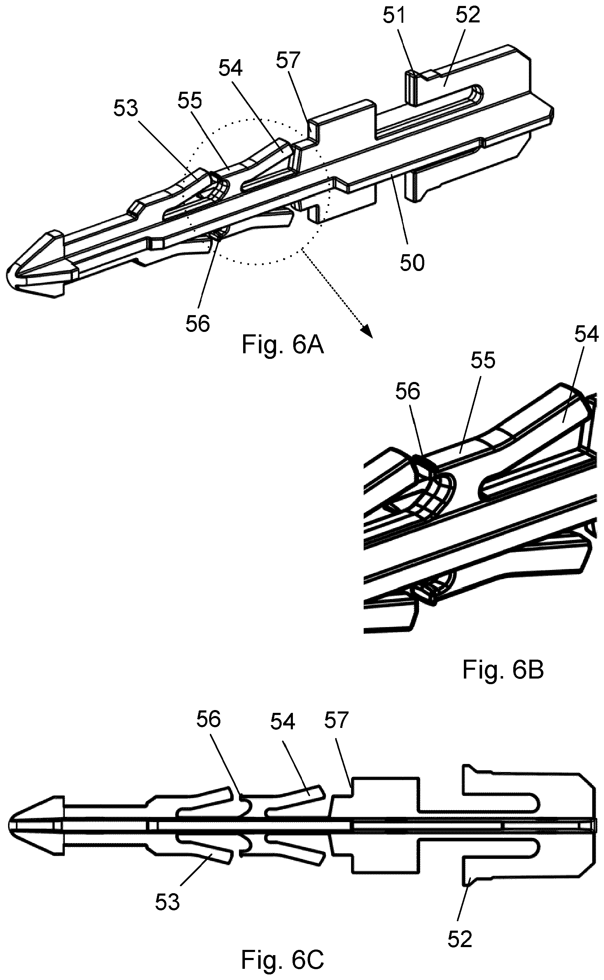

Figure 6A**-C** disclose the piston rod 50 which proximally carries the injection button 40 which is disclosed in detail in **figure 7****.**

The injection button 40 disclosed in figure 7 is provided with a number of radial openings 41 which engages with a similar number of radial snaps 51 on the piston rod 50 such that the injection button 40 and the piston rod 10 moves axially in unison. These snaps 51 are carried on a plurality of flexible arms 52 such that the piston rod 50 and the injection button 40 can easily be click-fitted together. Alternatively, the injection button 40 and the piston rod 50 can be moulded as one unitary element. The injection button 40 is further provided with circular ridges 42, 43 which guides the injection button 40 against the inner surface of the housing structure 1. Alternatively, these raised ridges 42, 43 could be provided as knobs or a similar support structure. Proximally, the injection button 40 is preferably provided with a concave surface to accommodate the finger of the user during injection.

Further, the piston rod 50 disclosed is provided with a first set 53 of flexible radial arms and a second set 54 of flexible radial arms. The second set 54 of flexible radial arms is on an outer surface provided with flat surfaces 55 which in one example each distally terminates in radial gripping teeth 56. This is best seen in the enlarged view in figure 6B. These flat surfaces are in a further example disclosed in figure 4C-D replaced by a set of further radial arms 58 which could carry the radial gripping teeth 56.

When the piston rod 50 is mounted in the nut element 60 as seen in figure 3, the flat surfaces 55 of the second set 54 of radial arms lies against the recessed inner surface 63 of the nut element 60. In this position, the radial gripping teeth 56 grips against the angled front 64 such that the position of the piston rod 50 relatively to the nut element 60 is fixed. This defines the start position of the piston rod 50 as will be explained. However, the start position is not necessarily physically positioned at the point of engagement, but the point of engagement defines the start position of the piston rod. Further, a radial stop surface 57 is provided on the piston rod 50 which defines the stop position and hence the possible length of the travel distance (TD) and hence the volume to be ejected as will be explained.

Alternatively, the piston rod 50 can be moulded without the radial gripping teeth 56. In such embodiment, the proximal end of the first set 53 of radial arms engage the angled front 64 to define the start position of the piston rod 50. Hence, distally the piston rod 50, preferably with the piston rod foot 45, abuts the plunger 12 inside the cartridge 10 and the proximal end of the first set 53 of the radial arms engages the angled front 64 on the nut element 60 thus defining the start position of the piston rod 50. Since temperature changes in the liquid drug can alter the position of the plunger 12 during storage, a little clearance between the proximal end of the first set 53 of arms and the angled front 64 of the nut element 60 is allowed for without changing the fact that the piston rod 50 (and/or foot 45) abuts the plunger 12 in the adjusted start position as will be explained.

In the latter example disclosed in figure 4C-D, the angled front 64 is preferably made with a radial slope of 90 degrees or a slope slightly towards the proximal end. This would make it almost impossible for the proximal end of the first set 53 of radial arms to slide proximally out of engagement with the nut element 60. Hence, the piston rod 50 would follow the nut element 60 in the proximal direction as the nut element 60 is rotated relatively to the housing structure 1 in the threaded connection 5, 65 and once the piston rod 50 (or foot 45) abuts the plunger 12 no further advancement of the nut element 60 would be possible.

In the example disclosed in figure 4C-D, the piston rod 50 could be moulded without the radial gripping teeth 56 such that the further radial arms 58 frictionally engages the recessed inner surface 63 to thereby define the stating point of the piston rod movement

The two sets of flexible radial arms 53, 54 can comprise any number of flexible arms in each set. However, in the disclosed example two flexible arms are provided in each set of radial arms 53, 54. As indicated in the figures, the flexible radial arms 53, 54 are flexible in a direction allowing only movement of the piston rod 50 toward the distal end of the housing structure 1. Once the piston rod 50 is mounted in the nut element 60 as disclosed in figure 3, the first set of arms 53 prevents the piston rod 50 from movement in the proximal direction by being blocked by the nut element 60.

In figure 3, the piston rod 10 is disclosed being positioned in the start position relatively to the housing structure 1. In this position, the piston rod 50 (and/or foot 45) abuts the plunger 12. The travel distance (TD), being the distance that the piston rod 50 (and hence the injection button 40) is allowed to move relatively to the housing structure 1 and thus the cartridge 10, which is secured at least axially to the housing structure 1, is also indicated in figure 3. The axial position of this travel distance (TD) in relation to the housing structure 1 can be adjusted by adjusting the position of the nut element 60 as will be explained in the following.

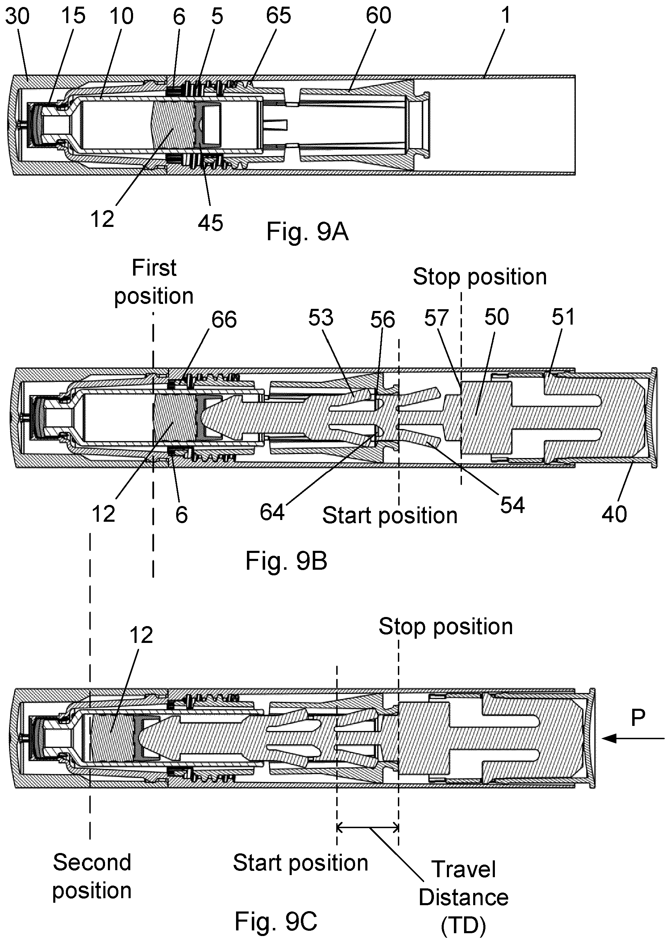

The start position as physically shown in figure 9B-C is located at the most proximal end of the nut element 60. Hence, the actual physical position is determined by the design of the nut element 60. However, independently of the design of the nut element 60, the start position of the piston rod 50 is the position wherein the piston rod 50 (and/or the foot 45) abuts the plunger 12.

The travel distance (TD) thus expresses the distance the piston rod 50 is able to move from its start position to its stop position. This length of the travel distance (TD) thus being a fixed distance, however, the physical position of the travel distance (TD) can be changed by adjusting the position of the nut element 60 relatively to the housing structure 1 in dependency of the position of the plunger 12 inside the individually filled cartridge 10.

### Assembly of the Injection Device:

When filling liquid drug into the individual cartridge 10 different tolerances apply. There are tolerances on the volume of liquid drug filled into the cartridge 10 from the filling line and there are tolerances on the physical dimensions on the cartridge 10. Filling of a cartridge 10 is usually done by filling the liquid drug into the cartridge 10 from the distal end of the cartridge 10. The plunger 12 is thus positioned in the distal part of the cartridge 10 prior to filling and during filling the plunger 12 moves proximally to the position wherein the correct volume of drug has been filled into the cartridge 10. Once filled, the distal end of the cartridge is sealed by the septum 11. Hence, due to the various tolerances, the end position of the plunger 12 when filled will vary from cartridge 10 to cartridge 10.

Further, as cartridges 10 are usually formed from glass the physical tolerances are relatively large. The different tolerances result in a slightly different axial position of the plunger 12 inside the individual cartridge 10 after filling the liquid drug into the cartridge 10.

After the cartridge 10 is filled with liquid drug, the cartridge 10 is pushed into the housing structure 1 in the distal direction such that the metal bend 15 on the cartridge 10 are press fitted into the crush ribs 7 in the housing structure 1 as disclosed in **figure 9A** or alternatively pressed into the housing structure by the crush flanges 67 provided inside the nut element 60 once the nut element 60 is positioned. In the final position, the cartridge 10 is firmly secured to the housing structure 1 at least axially.

When the cartridge 10 is placed and secured in the housing structure 1, the position of the plunger 12 is electronical measured by a computerized sensor system such that the exact position of the plunger 12 inside each individual cartridge 10 is registered.

When the position of the plunger 12 is measured, the nut element 60 is screwed into the thread 5 inside the housing structure 1 to a position wherein the piston rod 50 (when mounted) or preferably the piston rod foot 45 engages the plunger 12. The axial position of the nut element 60 is thus individually adjustable to an adjusted position during assembly of the injection device. The adjusted position being the position wherein the piston rod 50 (and/or foot 45) abuts the plunger 50 which is also the start position for the piston rod movement.

Figure 9A discloses the position of the nut element 60 prior to the thread segments 65 engages with the thread 5 in the housing structure 1. In this position, the ratchet carriers 66 has not yet engaged with the axial ribs 6 of the internal toothed ring in the housing structure 1.

When the nut element 60 has been rotated to its adjusted position, the piston rod 50 is pushed into the nut element 60 to a position wherein the first set 53 or radial arms or the gripping arms 56 engages the angled front 64. This engagement defines the start position of the travel distance (TD). As best seen in figure 3 and in figure 9B, the start position, all though defined by the engagement between the first set 53 of radial arms and the angled front or the gripping arms 56 and the angled front 64 is physically positioned at the proximal end of the proximal flange 68 of the nut element 60.

Alternatively, the piston rod 50 can first be positioned in the releasable secured position in nut element 60 before the nut element 60 is screwed into the housing structure 1 and the nut element 60 could be rotated until the piston rod 50 (or the piston rod foot 45) abuts the plunger 12 inside the cartridge 10.

In the disclosed example, the nut element 60 is provided with a number of ratchet carriers 66 which engages the axial ribs 6 inside the housing structure 1 such that the nut element 60 can only be rotated in one rotational direction in the thread connection between the thread 5 inside the housing structure 1 and the thread segments 65 provided on the nut element 60.

In one example the pitch of the thread 5 in the housing structure 1 and the pitch of the thread segments 65 on the nut element 60 can be e.g. 2 mm such that the nut element 60 is moved 2 mm in the distal direction per revolution. In such construction when e.g. 40 axial ribs 6 are provided inside the housing structure 1, the nut element 60 can be moved forward in increments of 0,05 mm (2 mm advancement to 40 ribs per revolution).

The allowed rotational direction being the one which moves the nut element 60 in the distal direction. When the nut element 60 has reached its adjusted position, it is thus not possible to rotate the nut element 60 in the proximal direction, as the ratchet interface 6, 66 only allows for rotation in one direction. In an alternative example, the nut element 60 can be irreversible secured to the housing structure 1 in the adjusted position preferably by welding the nut element 60 to the housing structure 1 e.g. by using laser welding. For this purpose, the housing structure 1 can be provided with a transparent area, an opening or the like such that the energy from a laser beam can be delivered to the area wherein the nut element 60 and the housing structure 1 has a physical point of contact.

Once the nut element 60 is positioned in the adjusted position, the piston rod 50 is inserted as disclosed in **figure 9B****.** In the adjusted position, the first set 53 of radial arms or the radial gripping teeth 56 on the piston rod 50 grips behind the angled front 64 inside the nut element 60 which defines the start position as indicated in figure 9B. However, due the axial extension existing between the angled front 64 and the proximal flange 68 of the nut element 60, the start position is physically positioned at the proximal end of the proximal flange 68 but defined by the engagement the first set 53 of radial arms and the angled front 64 or the gripping arms 56 and the angled front 64.

The starting point of the piston rod movement could also be defined by the frictional engagement of the further radial arms 58 and the recessed inner surface 63 as disclosed in figure 4C-D.

When the nut element 60 is in the adjusted position and the piston rod 50 is mounted in the start position, the distal end of the piston rod 50 preferably has physical contact with the proximal backside of the plunger 12 such that there is no physical distance between the piston rod 50 and the plunger 12. However, some slack is allowed due to temperature variations.

In the disclosed example a separate piston rod foot 45 is provided which can be inserted into the cartridge 10 prior to inserting the piston rod 50. When such piston rod foot 45 is used, the physical contact is between the piston rod foot 45 and the plunger 12.

When the piston rod 50 has been inserted as disclosed in figure 9B, the first set 53 of radial arms is positioned distal to the angled front 64 and are flexed towards the inner surface of the nut element 60 to thereby guide and stabilize the axial movement of the piston rod 50 during injection. The first set 53 of radial arms also prevents the piston rod 50 from movement in the proximal direction all though the piston rod 50 can be allowed to move a minor distance should the liquid drug inside the cartridge 10 expand due to temperature variations. However, it is not possible for a user to move the piston rod 50 in the proximal direction to thereby disassemble the injection device.

Once the injection device is assembled as disclosed in figure 9B, the injection device is ready to use and is thus sterilized and individually packed in a sterile wrapping and delivered to the end user.

When injecting, the user applies a pressure onto the injection button 40. This is indicated by the arrow "P" in **figure 9C****.** This pressure moves the injection button 40 and thereby the piston rod 50 in the distal direction. The axial movement of the piston rod 50 is possible until the stop surface 57 on the piston rod 50 abut the proximal end of the nut element 50 as disclosed in figure 9C. When the piston rod 50 enters into the stop position, the second set 54 of flexible arms engages against the angled surface 64 such that the piston rod 50 cannot be moved in the proximal direction.

The axial movement of the piston rod 50 from the start position to the stop position as indicated on figure 9B-C determines the distance the piston rod 50 is able to travel in one single stroke. The position of this travel distance (TD) relatively to the housing structure 1 and the cartridge 10 is adjustable by adjusting the position of the nut element 60 relatively to the housing structure 1. Preferably, the position of the travel distance (TD) is adjusted such that the piston rod 50 is in physical contact with the plunger 12 in the start position. Having no air gap between the piston rod 50 (and/or the foot 45) and the plunger 12 secures that the full stroke length of the piston rod 50 is transformed one-to-one to plunger movement.

The volume expelled during injection is determined by the first position of the plunger 12 prior to injection as disclosed in figure 9B and the second position of the plunger 12 following an injection as disclosed in figure 9C in combination with the inner dimensions of the individual cartridge 10.

When the piston rod 50 (or the piston rod foot 45) initially abuts the plunger 12 in the first position, the travel distance (TD) of the piston rod 50 will be transformed to a similar movement of the plunger 12 inside the cartridge 10 hence the length of the travel distance (TD) i.e. the distance between the start position and the stop position of the piston rod 50 and the length between the first position and the second position of the plunger 12 will be the same.

Some preferred embodiments have been shown in the foregoing, but it should be stressed that the invention is not limited to these but may be embodied in other ways within the subject matter defined in the following claims. The important issue reflected in the claims is to avoid that an air gap exist between the plunger and the piston rod foot after assembly, such that a part of the travel distance (TD) is consumed by this air gap and hence not fully transferred to plunger movement.

### List of Parts:

| | | | | | |
|---|---|---|---|---|---|
| 1 | Housing Structure | 31 | Textured Area | 61 | Support Flanges |
| 2 | Needle Interface | 32 | Ribs | 62 | Radial Opening |
| 3 | Protrusion | | | 63 | Recessed Inner Surface |
| 4 | Window | | | 64 | Angled Surface |
| 5 | Inner Thread | | | 65 | Outer Thread |
| 6 | Ribs | | | 66 | Ratchet Carrier, Flexible |
| 7 | Crush Ribs | | | 67 | Crush Flange |
| 8 | Longitudinal Ribs | | | 68 | Proximal Flange |
| 9 | Anti-Roll Feature | | | 69 | Friction Knobs |
| 10 | Cartridge | 40 | Injection Button | | |
| 11 | Septum | 41 | Radial Openings | | |
| 12 | Plunger | 42 | Circular Ridges | | |
| 13 | Distal Surface | 43 | Circular Ridges | | |
| 14 | Inner Surface | | | | |
| 15 | Metal Cap | 45 | Piston Rod Foot | | |
| 16 | Neck | | | | |
| | | | | | |
| | | | | | |
| | | | | | |
| 20 | Needle Assembly | 50 | Piston Rod | | |
| 21 | Cannula | 51 | Snaps | | |
| 22 | Hub | 52 | Flexible Arms | | |
| 23 | Proximal Part | 53 | First Set | | |
| 24 | Distal Point | 54 | Second Set | | |
| 25 | Enclosure | 55 | Flat surface | | |
| 26 | Tower | 56 | Gripping Arms | | |
| | | 57 | Radial Stop Surface | | |
| | | 58 | Small Radial Arms | | |
| | | | | | |
| 30 | Protective Cap | 60 | Nut Element | | |

## Claims

1. A pre-filled single dose injection device for delivering one single fixed dose of a predetermined volume of a liquid drug from an encapsulated cartridge, comprising:
a housing structure (1) securing the cartridge (10) containing the liquid drug which cartridge (10) is embedded in and axially locked to the housing structure (1) and which cartridge (10) has a distal end sealed by a pierceable septum (11) and a proximal end closed by a movable plunger (12) having a distal front surface (13),
a piston rod structure (50, 45) comprising a piston rod (50) and a piston rod foot (45), which is translationally movable in the distal direction in relation to the housing structure (1) to thereby move the plunger (12) in the distal direction inside the cartridge (10), the plunger (12) being movable from a first position to a second position, wherein
the predetermined volume of liquid drug to be ejected being defined by an inner surface (14) of the cartridge (10) and the length the distal front surface (13) of the plunger (12) is moved during movement of the plunger (12) from the first position to the second position, and
wherein a nut element (60) guiding the piston rod structure (50, 45) is adjustable coupled to the housing structure (1),
**Characterized in that**
the piston rod structure (50, 45) and the nut element (60) are provided with engagement means (53, 56, 58; 63, 64) for releasable securing the piston rod structure (50, 45) to the nut element (60) in a start position of the piston rod structure (50, 45) wherein the piston rod structure (50, 45) abuts the plunger (12) at least in use,
and wherein a stop position of the piston rod structure is defined by stop means (57) on the piston rod (50) operationally engaging the nut element (60), such that a travel distance (TD) of the piston rod structure (50, 45) is defined as the axial distance the piston rod structure (50, 45) is translationally movable when moving the plunger (12) from the start position to the stop position during dose ejection, and
wherein the axial position of the nut element (60) relatively to the housing structure (1) is individually adjustable for each individual injection device during assembly of the individual injection device to thereby adjust the axial position of the travel distance (TD) of the piston rod structure (50, 45) to an adjusted position in dependency of the position of the plunger (12) in the individual cartridge (10).

2. A single dose injection device according to claim 1, wherein the engaging means (53, 64) between the piston rod (50) and the nut element (60) comprises a number of radial arms (53) provided on either the piston rod (50) or the nut element (60) engaging the other of the piston rod (50) or the nut member (60).

3. A single dose injection device according to claim 2, wherein the radial arms (53) are provided on the piston rod (50) and the nut element (60) is provided with an angled surface (64) which is engaged by the radial arms (53) when the piston rod (50) is in its start position.

4. A single dose injection device according to claim 1, wherein the engagement means (58, 63) between the piston rod (50) and the nut element (60) comprises a number of further radial arms (58) provided on the piston rod (50) engaging a recessed inner surface (63) on the nut element (60) in the start position of the piston rod (50).

5. A single dose injection device according to claim 1, wherein the engaging means (56, 64) between the piston rod (50) and the nut element (60) comprises a number of radial gripping teeth (56) provided on either the piston rod (50) or the nut element (60) engaging the other of the piston rod (50) or the nut member (60).

6. A single dose injection device according to claim 5, wherein the radial gripping teeth (56) are provided on the piston rod (50) and the nut element (60) is provided with an angled surface (64) which is engaged by the radial gripping teeth (56) when the piston rod (50) is in its start position.

7. A single dose injection device according to any of the previous claims, wherein the nut element (60) proximally is provided with a proximal flange (68) which physically defines the start position for the piston rod (50) and engages with the stop means (57) on the piston rod (50) in the stop position.

8. A single dose injection device according to anyone of the preceding claims, wherein the nut element (60) is axially adjusted in relation to the housing structure (1) in a threaded connection (5, 65).

9. A single dose injection device according to claim 8, wherein the housing structure (1) is provided with a thread (5) on an inner surface engaging a corresponding thread (65) on an outer surface of the nut element (60).

10. A single dose injection device according to claim 9, wherein flanges of the threads (5, 65) are provided with axial friction knobs (69).

11. A single dose injection device according to claim 8, 9 or 10, wherein a one-way ratchet interface (6, 66) is provided between the nut element (60) and the housing structure (1).

12. A single dose injection device according to any of the previous claims, wherein one of the nut element (60) or the housing structure (1) are provided with a flexible ratchet carrier (66) and the other of the nut element (60) and the housing structure (1) are provided with an axial rib structure (6).

13. A single dose injection device according to anyone of the preceding claims, wherein the nut element (60) and the housing structure (1) are irreversible connected in the assembled state.

14. A method of assembling a pre-filled single dose injection device for delivering one single fixed dose of a predetermined volume of a liquid drug from an encapsulated cartridge, comprising:
a housing structure (1) securing the cartridge (10) containing the liquid drug which cartridge (10) is embedded in and axially locked to the housing structure (1) and which cartridge (10) has a distal end sealed by a pierceable septum (11) and a proximal end closed by a movable plunger (12) having a distal front surface (13),
a piston rod structure (50, 45) comprising a piston rod (50) and a piston rod foot (45), which is translationally movable in the distal direction in relation to the housing structure (1) to thereby move the plunger (12) in the distal direction inside the cartridge (10), the plunger (12) being movable from a first position to a second position, wherein
the predetermined volume of liquid drug to be ejected being defined by an inner surface (14) of the cartridge (10) and the length the front surface (13) of the plunger (12) is moved during movement of the plunger (12) from the first position to the second position, and
wherein a nut element (60) guiding the piston rod (50) is adjustable coupled to the housing structure (1),
**Characterized in that**,
the piston rod structure (50, 45) and the nut element (60) are provided with engagement means (53, 56, 58; 63, 64) for releasable securing the piston rod structure (50, 45) to the nut element (60) in a start position of the piston rod structure (50, 45) wherein the piston rod structure (50, 45) abuts the plunger (12),
and wherein a stop position of the piston rod structure (50, 45) is defined by stop means (57) on the piston rod (50) operationally engaging the nut element (60), such that a travel distance (TD) of the piston rod structure (50, 45) is defined as the axial distance the piston rod structure (50, 45) is translationally movable when moving the plunger (12) from the start position to the stop position during dose ejection, and
wherein the axial position of the nut element (60) relatively to the housing structure (1) is individually adjustable for each individual injection device during assembly of the individual injection device to thereby adjust the axial position of the travel distance (TD) of the piston rod structure (50, 45) to an adjusted position in dependency of the position of the plunger (12) in the individual cartridge (10),
wherein the method comprises the step of individually adjusting the axial position of the nut element (60) for each individual injection device during assembly to thereby adjust the axial position of the travel distance (TD) of the piston rod structure (50, 45) relatively to the housing structure (1) to an adjusted position wherein the piston rod structure (50, 45) abuts the plunger (12).

15. A plurality of single dose injection devices for delivering a predetermined volume of a liquid drug according to any of the claims 1 to 13 and wherein each single dose injection device in the plurality has been individually assembled and adjusted according to the method of claim 14.

## Patentansprüche

1. Vorgefüllte Einzeldosis-Injektionsvorrichtung zum Abgeben einer einzelnen festgelegten Dosis eines vorbestimmten Volumens eines flüssigen Arzneimittels aus einer eingekapselten Kartusche, umfassend:
eine Gehäusestruktur (1), die die Kartusche (10), die das flüssige Arzneimittel enthält, sichert, wobei die Kartusche (10) in die Gehäusestruktur (1) eingebettet und axial mit dieser verriegelt ist, und wobei die Kartusche (10) ein distales Ende, das durch ein durchstechbares Septum (11) abgedichtet ist, und ein proximales Ende, das durch einen beweglichen Kolben (12) mit einer distalen Stirnfläche (13) verschlossen ist, aufweist,
eine Kolbenstangenstruktur (50, 45), die eine Kolbenstange (50) und einen Kolbenstangenfuß (45) umfasst, der in der distalen Richtung in Bezug auf die Gehäusestruktur (1) translatorisch bewegbar ist, um dadurch den Kolben (12) in die distale Richtung innerhalb der Kartusche (10) zu bewegen, wobei der Kolben (12) von einer ersten Position in eine zweite Position bewegbar ist, wobei
das vorbestimmte Volumen des auszustoßenden flüssigen Arzneimittels durch eine Innenfläche (14) der Kartusche (10) und die durch die Länge definiert ist, um die die distale Stirnfläche (13) des Kolbens (12) während der Bewegung des Kolbens (12) von der ersten Position in die zweite Position bewegt wird, und
wobei ein Mutterelement (60), das die Kolbenstangenstruktur (50, 45) führt, einstellbar mit der Gehäusestruktur (1) gekoppelt ist,
**dadurch gekennzeichnet, dass**
die Kolbenstangenstruktur (50, 45) und das Mutterelement (60) mit Eingriffsmitteln (53, 56, 58; 63, 64) zum lösbaren Sichern der Kolbenstangenstruktur (50, 45) an dem Mutterelement (60) in einer Ausgangsposition der Kolbenstangenstruktur (50, 45) vorgesehen sind, wobei die Kolbenstangenstruktur (50, 45) mindestens in Gebrauch an dem Kolben (12) anliegt,
und wobei eine Anschlagposition der Kolbenstangenstruktur durch Anschlagmittel (57) an der Kolbenstange (50) definiert ist, die mit dem Mutterelement (60) in Wirkeingriff stehen, sodass eine Wegstrecke (TD) der Kolbenstangenstruktur (50, 45) als der axiale Abstand definiert ist, um den die Kolbenstangenstruktur (50, 45) translatorisch bewegbar ist, wenn der Kolben (12) während des Dosisausstoßes von der Ausgangsposition in die Anschlagposition bewegt wird, und
wobei die axiale Position des Mutterelements (60) relativ zu der Gehäusestruktur (1) für jede individuelle Injektionsvorrichtung während der Montage der individuellen Injektionsvorrichtung individuell einstellbar ist, um dadurch die axiale Position der Wegstrecke (TD) der Kolbenstangenstruktur (50, 45) in Abhängigkeit von der Position des Kolbens (12) in der individuellen Kartusche (10) auf eine eingestellte Position einzustellen.

2. Einzeldosis-Injektionsvorrichtung nach Anspruch 1, wobei die Eingriffsmittel (53, 64) zwischen der Kolbenstange (50) und dem Mutterelement (60) eine Anzahl radialer Arme (53) aufweisen, die entweder an der Kolbenstange (50) oder dem Mutterelement (60) vorgesehen sind und in das andere der Kolbenstange (50) oder des Mutterelements (60) eingreifen.

3. Einzeldosis-Injektionsvorrichtung nach Anspruch 2, wobei die radialen Arme (53) an der Kolbenstange (50) vorgesehen sind und das Mutterelement (60) mit einer schrägen Fläche (64) vorgesehen ist, in die die radialen Arme (53) eingreifen, wenn sich die Kolbenstange (50) in ihrer Ausgangsposition befindet.

4. Einzeldosis-Injektionsvorrichtung nach Anspruch 1, wobei die Eingriffsmittel (58, 63) zwischen der Kolbenstange (50) und dem Mutterelement (60) eine Anzahl weiterer radialer Arme (58) aufweisen, die an der Kolbenstange (50) vorgesehen sind und in der Ausgangsposition der Kolbenstange (50) in eine vertiefte Innenfläche (63) an dem Mutterelement (60) eingreifen.

5. Einzeldosis-Injektionsvorrichtung nach Anspruch 1, wobei die Eingriffsmittel (56, 64) zwischen der Kolbenstange (50) und dem Mutterelement (60) eine Anzahl radialer Greifzähne (56) aufweisen, die entweder an der Kolbenstange (50) oder dem Mutterelement (60) vorgesehen sind und in das andere der Kolbenstange (50) oder des Mutterelements (60) eingreifen.

6. Einzeldosis-Injektionsvorrichtung nach Anspruch 5, wobei die radialen Greifzähne (56) an der Kolbenstange (50) vorgesehen sind und das Mutterelement (60) mit einer schrägen Fläche (64) vorgesehen ist, in die die radialen Greifzähne (56) eingreifen, wenn sich die Kolbenstange (50) in ihrer Ausgangsposition befindet.

7. Einzeldosis-Injektionsvorrichtung nach einem der vorhergehenden Ansprüche, wobei das Mutterelement (60) proximal mit einem proximalen Flansch (68) vorgesehen ist, der physisch die Ausgangsposition für die Kolbenstange (50) definiert und in der Anschlagposition in die Anschlagmittel (57) an der Kolbenstange (50) eingreift.

8. Einzeldosis-Injektionsvorrichtung nach einem der vorhergehenden Ansprüche, wobei das Mutterelement (60) in einer Gewindeverbindung (5, 65) in Bezug auf die Gehäusestruktur (1) axial eingestellt ist.

9. Einzeldosis-Injektionsvorrichtung nach Anspruch 8, wobei die Gehäusestruktur (1) an einer Innenfläche mit einem Gewinde (5) vorgesehen ist, das in ein entsprechendes Gewinde (65) an einer Außenfläche des Mutterelements (60) eingreift.

10. Einzeldosis-Injektionsvorrichtung nach Anspruch 9, wobei Flansche der Gewinde (5, 65) mit axialen Reibungsnoppen (69) vorgesehen sind.

11. Einzeldosis-Injektionsvorrichtung nach Anspruch 8, 9 oder 10, wobei eine Einweg-Ratschen-Schnittstelle (6, 66) zwischen dem Mutterelement (60) und der Gehäusestruktur (1) vorgesehen ist.

12. Einzeldosis-Injektionsvorrichtung nach einem der vorhergehenden Ansprüche, wobei eines des Mutterelements (60) oder der Gehäusestruktur (1) mit einem flexiblen Ratschenträger (66) vorgesehen sind und das andere des Mutterelements (60) und der Gehäusestruktur (1) mit einer axialen Rippenstruktur (6) vorgesehen sind.

13. Einzeldosis-Injektionsvorrichtung nach einem der vorhergehenden Ansprüche, wobei das Mutterelement (60) und die Gehäusestruktur (1) in zusammengebautem Zustand irreversibel verbunden sind.

14. Verfahren zum Zusammenbauen einer vorgefüllten Einzeldosis-Injektionsvorrichtung zum Abgeben einer einzelnen festgelegten Dosis eines vorbestimmten Volumens eines flüssigen Arzneimittels aus einer eingekapselten Kartusche, umfassend:
eine Gehäusestruktur (1), die die Kartusche (10), die das flüssige Arzneimittel enthält, sichert, wobei die Kartusche (10) in die Gehäusestruktur (1) eingebettet und axial mit dieser verriegelt ist, und wobei die Kartusche (10) ein distales Ende, das durch ein durchstechbares Septum (11) abgedichtet ist, und ein proximales Ende, das durch einen beweglichen Kolben (12) mit einer distalen Stirnfläche (13) verschlossen ist, aufweist,
eine Kolbenstangenstruktur (50, 45), umfassend eine Kolbenstange (50) und einen Kolbenstangenfuß (45), der in der distalen Richtung in Bezug auf die Gehäusestruktur (1) translatorisch bewegbar ist, um dadurch den Kolben (12) in die distale Richtung innerhalb der Kartusche (10) zu bewegen, wobei der Kolben (12) von einer ersten Position in eine zweite Position bewegbar ist, wobei
das vorbestimmte Volumen des auszustoßenden flüssigen Arzneimittels durch eine Innenfläche (14) der Kartusche (10) und durch die Länge definiert ist, um die die Stirnfläche (13) des Kolbens (12) während der Bewegung des Kolbens (12) von der ersten Position in die zweite Position bewegt wird, und
wobei ein Mutterelement (60), das die Kolbenstange (50) führt, einstellbar mit der Gehäusestruktur (1) gekoppelt ist,
**dadurch gekennzeichnet, dass**
die Kolbenstangenstruktur (50, 45) und das Mutterelement (60) mit Eingriffsmitteln (53, 56, 58; 63, 64) zum lösbaren Sichern der Kolbenstangenstruktur (50, 45) an dem Mutterelement (60) in einer Ausgangsposition der Kolbenstangenstruktur (50, 45) vorgesehen sind, wobei die Kolbenstangenstruktur (50, 45) an dem Kolben (12) anliegt,
und wobei eine Anschlagposition der Kolbenstangenstruktur (50, 45) durch Anschlagmittel (57) an der Kolbenstange (50) definiert ist, die mit dem Mutterelement (60) in Wirkeingriff stehen, sodass eine Wegstrecke (TD) der Kolbenstangenstruktur (50, 45) als der axiale Abstand definiert ist, um den die Kolbenstangenstruktur (50, 45) translatorisch bewegbar ist, wenn der Kolben (12) während des Dosisausstoßes von der Ausgangsposition in die Anschlagposition bewegt wird, und
wobei die axiale Position des Mutterelements (60) relativ zu der Gehäusestruktur (1) für jede individuelle Injektionsvorrichtung während der Montage der individuellen Injektionsvorrichtung individuell einstellbar ist, um dadurch die axiale Position der Wegstrecke (TD) der Kolbenstangenstruktur (50, 45) in Abhängigkeit von der Position des Kolbens (12) in der individuellen Kartusche (10) auf eine eingestellte Position einzustellen,
wobei das Verfahren den Schritt des individuellen Einstellens der axialen Position des Mutterelements (60) für jede individuelle Injektionsvorrichtung während der Montage umfasst, um dadurch die axiale Position der Wegstrecke (TD) der Kolbenstangenstruktur (50, 45) in Bezug auf die Gehäusestruktur (1) in eine eingestellte Position einzustellen, in der die Kolbenstangenstruktur (50, 45) an dem Kolben (12) anliegt.

15. Mehrzahl von Einzeldosis-Injektionsvorrichtungen zum Abgeben eines vorbestimmten Volumens eines flüssigen Arzneimittels nach einem der Ansprüche 1 bis 13, wobei jede Einzeldosis-Injektionsvorrichtung in der Mehrzahl individuell zusammengebaut und gemäß dem Verfahren nach Anspruch 14 eingestellt wurde.

## Revendications

1. Dispositif d'injection à dose unique pré-rempli destiné à délivrer une dose fixe unique d'un volume prédéterminé d'un médicament liquide à partir d'une cartouche encapsulée, comprenant :
une structure de boîtier (1) arrimant la cartouche (10) contenant le médicament liquide, laquelle cartouche (10) est encastrée dans la structure de boîtier (1) et verrouillée axialement à celle-ci, et laquelle cartouche (10) présente une extrémité distale scellée par un septum perçable (11) et une extrémité proximale fermée par un plongeur mobile (12) ayant une surface avant distale (13),
une structure de tige de piston (50, 45) comprenant une tige de piston (50) et un pied de tige de piston (45), qui est mobile en translation dans la direction distale par rapport à la structure de boîtier (1) pour déplacer ainsi le plongeur (12) dans la direction distale à l'intérieur de la cartouche (10), le plongeur (12) étant mobile depuis une première position jusqu'à une deuxième position, dans lequel
le volume prédéterminé de médicament liquide à éjecter étant défini par une surface intérieure (14) de la cartouche (10) et la longueur sur laquelle la surface avant distale (13) du plongeur (12) est déplacée pendant le déplacement du plongeur (12) depuis la première position jusqu'à la deuxième position, et
dans lequel un élément d'écrou (60) guidant la structure de tige de piston (50, 45) est accouplé de manière réglable à la structure de boîtier (1),
**caractérisé en ce que**,
la structure de tige de piston (50, 45) et l'élément d'écrou (60) sont munis de moyens de mise en prise (53, 56, 58 ; 63, 64) pour arrimer de manière libérable la structure de tige de piston (50, 45) à l'élément d'écrou (60) dans une position de départ de la structure de tige de piston (50, 45) dans lequel la structure de tige de piston (50, 45) vient en butée contre le plongeur (12) au moins en utilisation,
et dans lequel une position d'arrêt de la structure de tige de piston est définie par des moyens d'arrêt (57) sur la tige de piston (50) venant en prise de manière fonctionnelle avec l'élément d'écrou (60), de telle sorte qu'une distance de parcours (TD) de la structure de tige de piston (50, 45) est définie comme la distance axiale sur laquelle la structure de tige de piston (50, 45) est mobile en translation lors du déplacement du plongeur (12) depuis la position de départ jusqu'à la position d'arrêt pendant l'éjection de la dose, et
dans lequel la position axiale de l'élément d'écrou (60) par rapport à la structure de boîtier (1) est réglable individuellement pour chaque dispositif d'injection individuel pendant l'assemblage du dispositif d'injection individuel pour ainsi régler la position axiale de la distance de parcours (TD) de la structure de tige de piston (50, 45) à une position réglée en fonction de la position du plongeur (12) dans la cartouche (10) individuelle.

2. Dispositif d'injection à dose unique selon la revendication 1, dans lequel le moyen de mise en prise (53, 64) entre la tige de piston (50) et l'élément d'écrou (60) comprend un certain nombre de bras radiaux (53) prévus soit sur la tige de piston (50), soit sur l'élément d'écrou (60) mettant en prise l'autre parmi la tige de piston (50) ou l'élément d'écrou (60).

3. Dispositif d'injection à dose unique selon la revendication 2, dans lequel les bras radiaux (53) sont prévus sur la tige de piston (50) et l'élément d'écrou (60) est muni d'une surface inclinée (64) qui est mise en prise par les bras radiaux (53) lorsque la tige de piston (50) est dans sa position de départ.

4. Dispositif d'injection à dose unique selon la revendication 1, dans lequel le moyen de mise en prise (58, 63) entre la tige de piston (50) et l'élément d'écrou (60) comprend un certain nombre de bras radiaux supplémentaires (58) prévus sur la tige de piston (50) venant en prise avec une surface intérieure évidée (63) sur l'élément d'écrou (60) dans la position de départ de la tige de piston (50).

5. Dispositif d'injection à dose unique selon la revendication 1, dans lequel le moyen de mise en prise (56, 64) entre la tige de piston (50) et l'élément d'écrou (60) comprend un certain nombre de dents de préhension radiales (56) prévues soit sur la tige de piston (50), soit sur l'élément d'écrou (60) mettant en prise l'autre parmi la tige de piston (50) ou l'élément d'écrou (60).

6. Dispositif d'injection à dose unique selon la revendication 5, dans lequel les dents de préhension radiales (56) sont prévues sur la tige de piston (50) et l'élément d'écrou (60) est muni d'une surface inclinée (64) qui est mise en prise par les dents de préhension radiales (56) lorsque la tige de piston (50) est dans sa position de départ.

7. Dispositif d'injection à dose unique selon l'une quelconque des revendications précédentes, dans lequel l'élément d'écrou (60) est muni de manière proximale d'une bride proximale (68) qui définit physiquement la position de départ pour la tige de piston (50) et vient en prise avec le moyen d'arrêt (57) sur la tige de piston (50) dans la position d'arrêt.

8. Dispositif d'injection à dose unique selon l'une quelconque des revendications précédentes, dans lequel l'élément d'écrou (60) est réglé axialement par rapport à la structure de boîtier (1) dans une connexion filetée (5, 65).

9. Dispositif d'injection à dose unique selon la revendication 8, dans lequel la structure de boîtier (1) est munie d'un filetage (5) sur une surface intérieure venant en prise un filetage (65) correspondant sur une surface extérieure de l'élément d'écrou (60).

10. Dispositif d'injection à dose unique selon la revendication 9, dans lequel des brides des filetages (5, 65) sont munies de molettes de friction axiales (69).

11. Dispositif d'injection à dose unique selon la revendication 8, 9 ou 10, dans lequel une interface à cliquet unidirectionnel (6, 66) est prévue entre l'élément d'écrou (60) et la structure de boîtier (1).

12. Dispositif d'injection à dose unique selon l'une quelconque des revendications précédentes, dans lequel l'un parmi l'élément d'écrou (60) ou la structure de boîtier (1) est muni d'un support d'encliquetage flexible (66) et l'autre parmi l'élément d'écrou (60) et la structure de boîtier (1) est muni d'une structure de nervure axiale (6).

13. Dispositif d'injection à dose unique selon l'une quelconque des revendications précédentes, dans lequel l'élément d'écrou (60) et la structure de boîtier (1) sont connectés de manière irréversible dans l'état assemblé.

14. Procédé d'assemblage d'un dispositif d'injection à dose unique pré-rempli destiné à délivrer une dose fixe unique d'un volume prédéterminé d'un médicament liquide à partir d'une cartouche encapsulée, comprenant :
une structure de boîtier (1) arrimant la cartouche (10) contenant le médicament liquide, laquelle cartouche (10) est encastrée dans la structure de boîtier (1) et verrouillée axialement à celle-ci, et laquelle cartouche (10) présente une extrémité distale scellée par un septum perçable (11) et une extrémité proximale fermée par un plongeur mobile (12) ayant une surface avant distale (13),
une structure de tige de piston (50, 45) comprenant une tige de piston (50) et un pied de tige de piston (45), qui est mobile en translation dans la direction distale par rapport à la structure de boîtier (1) pour déplacer ainsi le plongeur (12) dans la direction distale à l'intérieur de la cartouche (10), le plongeur (12) étant mobile depuis une première position jusqu'à une deuxième position, dans lequel
le volume prédéterminé de médicament liquide à éjecter étant défini par une surface intérieure (14) de la cartouche (10) et la longueur sur laquelle la surface avant (13) du plongeur (12) est déplacée pendant le déplacement du plongeur (12) depuis la première position jusqu'à la deuxième position, et
dans lequel un élément d'écrou (60) guidant la tige de piston (50) est accouplé de manière réglable à la structure de boîtier (1),
**Caractérisé en ce que**,
la structure de tige de piston (50, 45) et l'élément d'écrou (60) sont munis de moyens de mise en prise (53, 56, 58 ; 63, 64) pour arrimer de manière libérable la structure de tige de piston (50, 45) à l'élément d'écrou (60) dans une position de départ de la structure de tige de piston (50, 45) dans lequel la structure de tige de piston (50, 45) vient en butée contre le plongeur (12),
et dans lequel une position d'arrêt de la structure de tige de piston (50, 45) est définie par des moyens d'arrêt (57) sur la tige de piston (50) venant en prise de manière fonctionnelle avec l'élément d'écrou (60), de telle sorte qu'une distance de parcours (TD) de la structure de tige de piston (50, 45) est définie comme la distance axiale sur laquelle la structure de tige de piston (50, 45) est mobile en translation lors du déplacement du plongeur (12) depuis la position de départ jusqu'à la position d'arrêt pendant l'éjection de la dose, et
dans lequel la position axiale de l'élément d'écrou (60) par rapport à la structure de boîtier (1) est réglable individuellement pour chaque dispositif d'injection individuel pendant l'assemblage du dispositif d'injection individuel pour ainsi régler la position axiale de la distance de parcours (TD) de la structure de tige de piston (50, 45) à une position réglée en fonction de la position du plongeur (12) dans la cartouche (10) individuelle,
dans lequel le procédé comprend l'étape consistant à régler individuellement la position axiale de l'élément d'écrou (60) pour chaque dispositif d'injection individuel pendant l'assemblage pour ainsi régler la position axiale de la distance de parcours (TD) de la structure de tige de piston (50, 45) par rapport à la structure de boîtier (1) à une position réglée, dans lequel la structure de tige de piston (50, 45) vient en butée contre le plongeur (12).

15. Pluralité de dispositifs d'injection à dose unique destinés à délivrer un volume prédéterminé d'un médicament liquide selon l'une quelconque des revendications 1 à 13 et dans laquelle chaque dispositif d'injection à dose unique parmi la pluralité a été assemblé et réglé individuellement selon le procédé de la revendication 14.
